# FASCICULE DE BREVET EUROPEEN

(11) **EP 1 584 340 B1**
(45) Date de publication et mention de la délivrance du brevet: **26.03.2008**
(21) Numéro de dépôt: 05290698.9
(22) Date de dépôt: 30.03.2005
(51) Int. Cl.: A61M 3/02

(54) **Appareil pour moucher les personnes**
Vorrichtung zum Nasenputzen
Apparatus for cleaning the nose

(30) Priorité: 06.04.2004 FR 0403577
(43) Date de publication de la demande: 12.10.2005
(73) Titulaire: Société Anonyme Monegasque Incomex, 98000 Monaco (MC)
(72) Inventeur: Gillo, Martin, 94130 Nogent sur Marne (FR); Blayn, Marie-Caroline, 94130 Nogent sur Marne (FR); Marquant, Pascal, 41100 Meslay (FR)
(74) Mandataire: Lavialle, Bruno François Stéphane

(56) Documents cités:
- EP-A- 0 243 261
- FR-A- 2 596 277
- FR-A- 2 605 227
- FR-A- 2 787 334
- US-A- 1 856 811
- US-A- 2 485 184
- US-A1- 2003 145 849

## Description

La présente invention concerne un appareil pour moucher les personnes et notamment les enfants en bas âge, un tel appareil étant alors couramment appelé "mouche-bébé".

La présente invention constitue plus particulièrement un perfectionnement de l'appareil faisant l'objet du brevet d'invention français No 2 562 424 déposé le 5 avril 1984.

On rappelle qu'un mouche-bébé comprend un corps délimitant un conduit reliant un embout nasal et un embout d'aspiration et est généralement destiné à être manipulé par un adulte pour extraire par aspiration des sécrétions encombrant le nez d'un enfant dans lequel est introduit l'embout nasal.

Pour faciliter l'évacuation des sécrétions hors du nez de l'enfant, il est connu de préalablement pulvériser à l'intérieur de celui-ci un liquide, généralement du sérum physiologique, qui fluidifie les sécrétions.

Le sérum physiologique est habituellement contenu dans une bouteille en matière plastique déformable qui comporte une portion sensiblement en forme d'embout nasal dont l'extrémité doit être sectionnée pour former un orifice de sortie du sérum physiologique. L'adulte introduit cet embout dans une narine de l'enfant et presse les flancs de la bouteille pour en chasser le sérum. Les sécrétions sont ensuite aspirées au moyen du mouche-bébé.

Un inconvénient de cette méthode est qu'elle nécessite d'introduire successivement, dans la narine de l'enfant, l'embout de la bouteille pour y pulvériser le sérum puis l'embout nasal pour aspirer les sécrétions. Or, il est fréquent que l'enfant se débatte lors de ces opérations, ce qui rend leur réalisation difficile contribuant ainsi à augmenter l'énervement de l'enfant. Par ailleurs, lorsque la pression exercée sur la bouteille pour la pulvérisation est relâchée alors que l'embout de la bouteille est encore dans une narine de l'enfant, une partie des sécrétions est aspirée dans la bouteille qui n'est alors plus utilisable.

Pour remédier à ces inconvénients, il est connu, notamment des documents FR-A-2 787 334 et FR-A-2 596 277, un appareil pour moucher comprenant un corps qui délimite un conduit principal ayant une extrémité pourvue d'un embout nasal et une extrémité opposée pourvue d'un moyen d'aspiration, et qui est associé à un conduit additionnel ayant une première extrémité débouchant dans l'embout nasal et une deuxième extrémité en communication avec un réservoir de sérum physiologique monté sur le corps et pourvu de moyens de propulsion du liquide fonctionnel depuis ledit réservoir jusqu'à l'embout nasal. Ces appareils limitent le nombre de manipulations pour instiller le sérum physiologique et aspirer les mucosités mais sont relativement complexes et/ou nécessitent d'être manipulés à deux mains à moins de recourir à des moyens motorisés de propulsion du sérum physiologique. Il est en outre connu du document US-A-1 856 811 un appareil analogue à ceux précités.

Un but de l'invention est de proposer un moyen simple et d'utilisation aisée permettant une aspiration efficace des sécrétions encombrant le nez d'un enfant en limitant le nombre de manipulations nécessaires pour y parvenir.

A cet effet, on prévoit, selon l'invention, un appareil pour moucher notamment un individu, comprenant un corps délimitant un conduit principal ayant une extrémité pourvue d'un embout nasal et une extrémité opposée pourvue d'un moyen d'aspiration, l'appareil comprenant un conduit additionnel ayant une première extrémité débouchant dans l'embout nasal et une deuxième extrémité en communication avec un réservoir de liquide fonctionnel monté sur le corps et agencé pour présenter un volume variable pour permettre une propulsion du liquide fonctionnel depuis ledit réservoir jusqu'à l'embout nasal, le conduit principal comportant entre ses deux extrémités un tronçon coudé sur une portion externe duquel est monté le réservoir.

Ainsi, une fois l'embout nasal introduit dans la narine de l'enfant, le liquide fonctionnel peut être pulvérisé et les sécrétions nasales aspirées. La réalisation de ces deux opérations ne nécessite qu'une introduction de l'embout nasal dans chaque narine de l'enfant. Ceci limite la durée du mouchage et l'inconfort de l'opération pour l'enfant. L'aménagement d'un tronçon coudé sur le corps et la fixation du réservoir à volume variable sur une portion externe du tronçon coudé permettent l'obtention d'une structure simple et compacte, manipulable d'une seule main par l'adulte qui peut ainsi maintenir l'enfant de son autre main sans jamais avoir à le lâcher. En outre, le réservoir est alors disposé dans une zone du corps qui n'est pas trop encombrée, ce qui facilite l'accès au réservoir et peut contribuer à équilibrer l'appareil.

Selon un mode de réalisation particulier du réservoir, le réservoir est de préférence conformé en un soufflet comportant un orifice de sortie qui est disposé selon un axe central du soufflet et qui est en communication avec la deuxième extrémité du conduit additionnel.

Les moyens de pulvérisation sont alors incorporés à la structure du réservoir puisqu'une pression sur le réservoir permet de diminuer le volume du réservoir et d'en expulser le liquide.

Selon un mode de réalisation particulier du conduit additionnel, le conduit additionnel s'étend dans le conduit principal et est de préférence formé par un tube rapporté de façon amovible dans le conduit principal.

Ceci améliore l'esthétique de l'appareil et confère à l'appareil une structure relativement simple. Le caractère amovible du tube facilite le nettoyage de l'appareil tandis que son positionnement à l'intérieur du conduit principal limite les risques de perte du conduit additionnel.

De préférence, le réservoir est monté de façon amovible sur le corps.

Il suffit alors de démonter le réservoir pour le remplir de liquide fonctionnel ou le nettoyer.

Avantageusement alors, le corps comprend un canal ayant une première extrémité dans laquelle débouche la deuxième extrémité du conduit additionnel et une deuxième extrémité débouchant à l'extérieur du corps par une tétine sur laquelle est emmanchée à force une extrémité ouverte du réservoir.

Ce mode de réalisation est particulièrement simple.

D'autres caractéristiques et avantages de l'invention ressortiront à la lecture de la description qui suit d'un mode de réalisation particulier non limitatif de l'invention.

Il sera fait référence aux dessins annexés, parmi lesquels :
- la figure 1 est une vue en perspective et en éclaté de l'appareil conforme à l'invention,
- la figure 2 est une vue schématique en coupe longitudinale de cet appareil,
- la figure 3 est une vue en coupe longitudinale d'un embout pour cet appareil, dans une première variante de réalisation de l'invention,
- la figure 4 est une vue en perspective d'un embout d'aspiration pour cet appareil, selon une deuxième variante de réalisation de l'invention.

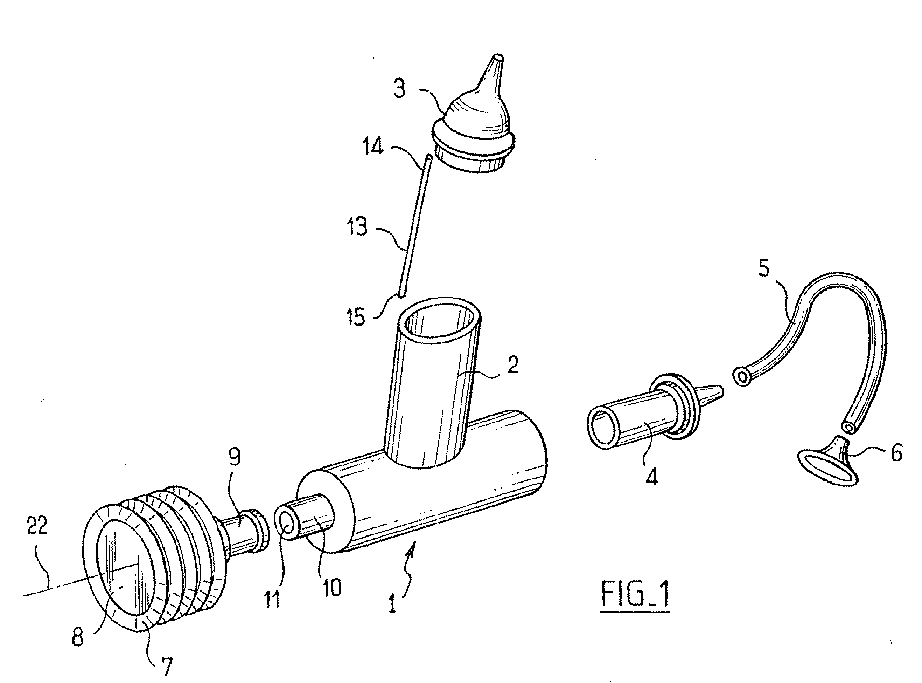

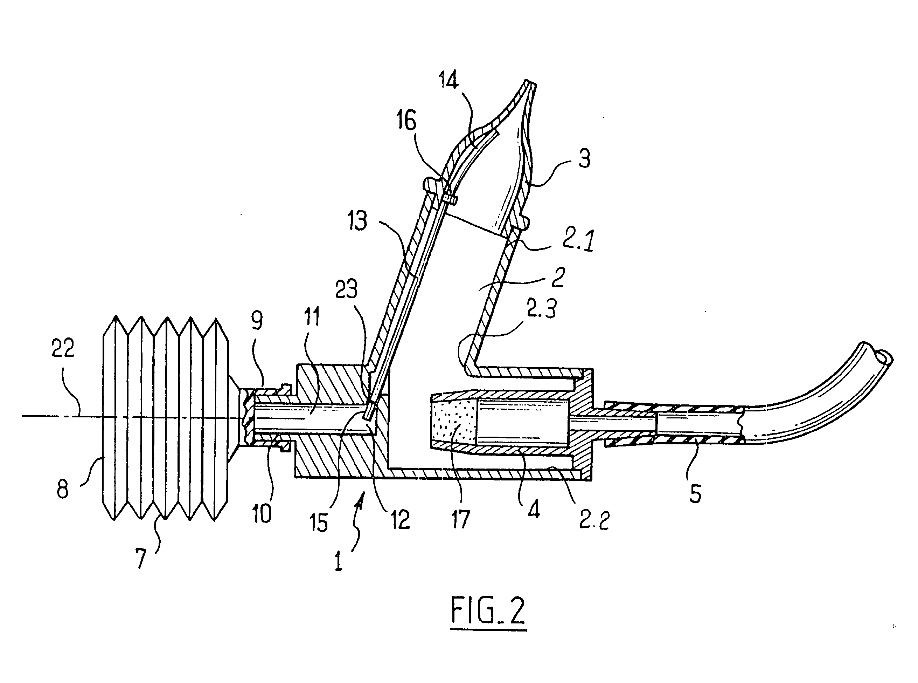

En référence aux figures 1 et 2, l'appareil de mouchage de l'invention comprend un corps généralement désigné en 1 délimitant un conduit principal 2, cylindrique, comportant une extrémité 2.1 à laquelle est fixé un embout nasal 3 et une extrémité 2.2 opposée recevant un insert 4 de raccordement d'un tuyau souple 5 à l'extrémité libre duquel est fixé un embout d'aspiration 6. Les deux extrémités 2.1, 2.2 sont reliées par un tronçon coudé 2.3 formant ici un angle aigu.

L'embout nasal 3 et l'insert 4 sont montés de façon amovible sur le corps 1 pour en faciliter le nettoyage. L'embout nasal 3 et l'insert 4 sont en l'espèce insérés légèrement à force dans les extrémités du conduit principal 2. L'embout nasal 3 a ici une forme classique en ogive.

L'embout d'aspiration 6 est ici destiné à être pincé entre les lèvres de l'adulte manipulant le mouche bébé.

La structure mentionnée ci-dessus est connue en elle-même et fait l'objet du brevet d'invention français No 2 562 424 précité.

Un réservoir généralement désigné en 7 est monté de manière amovible sur le corps 1. Le réservoir 7 a ici une forme en soufflet ayant une extrémité obturée 8 et une extrémité ouverte 9 pourvue d'un orifice de sortie disposé sur un axe central 22 du soufflet. Le réservoir 7 est ici sensiblement coaxial à l'insert 4. Le réservoir 7 est réalisé dans un matériau élastiquement déformable, ici en un élastomère thermoplastique.

L'extrémité ouverte 9 du réservoir 7 est emmanchée à force sur une tétine 10 qui est solidaire d'une partie externe du tronçon coudé 2.3 et qui s'étend ici dans l'axe de l'extrémité 2.2 du conduit principal 2 formé par le corps 1. La tétine 10 forme une extrémité d'un canal 11 qui est délimité par le corps 1 et qui possède une extrémité opposée 12 dans le conduit principal 2.

Un conduit secondaire 13 (ou additionnel) débouche dans l'extrémité 12 du canal 11. Le conduit secondaire 13 est formé par un tube qui est rapporté dans le conduit principal 2 et qui possède une première extrémité 14 débouchant dans l'embout nasal 3 et une deuxième extrémité 15 engagée légèrement à force dans un perçage 23 ménagé transversalement dans l'extrémité 12 du canal 11 pour être en communication avec ce dernier. L'extrémité 14 est fixée dans l'embout nasal 3 au voisinage et en retrait de l'orifice de celui-ci au moyen d'un clip ou d'un oeillet 16 qui est solidaire de l'embout nasal 3 et qui constitue un élément de maintien de l'extrémité 14 dans l'embout nasal 3.

Le fonctionnement de l'appareil va maintenant être décrit.

Préalablement à son utilisation, le réservoir 7 est déconnecté du corps 1 pour être rempli par son extrémité ouverte au moyen d'un liquide fonctionnel permettant ici la fluidification des sécrétions nasales. Le liquide utilisé est par exemple du sérum physiologique mais peut être également un autre liquide tel que de l'eau.

Après remplissage, le réservoir 7 est monté sur le corps 1 et l'adulte maintient d'une main le corps 1 de l'appareil de manière à avoir un doigt susceptible de presser le réservoir 7 selon une direction parallèle à l'axe central du réservoir 7. L'adulte introduit l'embout d'aspiration 6 dans sa bouche et introduit l'embout nasal 3 dans la narine du bébé.

L'adulte exerce une pression sur le réservoir 7 afin de pulvériser dans la narine du bébé le liquide fonctionnel.

Après cette pulvérisation, l'adulte aspire les sécrétions nasales qui seront récupérées dans le tronçon coudé du conduit principal 2 du corps 1.

Il est possible de prévoir un filtre 17 dans l'insert 4 à proximité du débouché du tuyau souple 5. Ce filtre 17 est par exemple un filtre en tissu ou en ouate, éventuellement imprégné d'un liquide antiseptique, qui permet le passage de l'air tout en retenant les sécrétions.

On notera que compte tenu du faible diamètre du conduit secondaire 13 et de la position en retrait de l'extrémité 14 de celui-ci dans l'embout nasal 3, les sécrétions ne peuvent pénétrer dans le conduit secondaire 13.

La position en retrait de l'extrémité 14 du conduit secondaire 13 par rapport à l'orifice de l'embout nasal 3 dans le mode de réalisation décrit a un autre avantage. Lorsque cesse la pression exercée sur le réservoir pour en expulser du liquide, le réservoir revient dans son état normal du fait de son élasticité de sorte que de l'air est aspiré vers le réservoir. L'extrémité 14 du conduit secondaire 13 étant en retrait par rapport à l'orifice de l'embout nasal 3, l'air aspiré provient essentiellement du corps 1 et non pas de la narine de l'enfant. Le risque d'une aspiration accidentelle des sécrétions dans le réservoir 7 est donc limité.

Bien entendu, l'invention n'est pas limitée au mode de réalisation décrit et on peut y apporter des variantes de réalisation sans sortir du cadre de l'invention tel que défini par les revendications.

Le liquide fonctionnel peut en outre avoir d'autres fonctions comme une fonction nettoyante, antiseptique, analgésique...

Le réservoir 7 peut être agencé différemment tout en ayant un volume variable. Le réservoir 7 peut ainsi comprendre un corps rigide recevant à coulissement un piston.

Par ailleurs, l'embout d'aspiration peut être raccordé à une pompe motorisée.

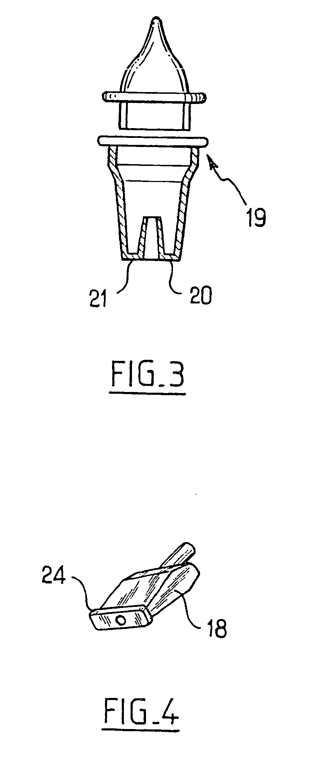

En variante et conformément à la figure 4, l'embout d'aspiration peut avoir la forme de l'embout d'aspiration 18 qui est réalisé en plastique rigide ou semi-rigide et qui est agencé pour être serré entre les dents, un bourrelet 24 venant en butée de la face arrière des dents pour éviter que l'embout d'aspiration 18 soit extrait accidentellement de la bouche de l'adulte manipulant l'appareil.

En variante également et conformément à la figure 3, l'embout nasal 3 peut être remplacé par un embout nasal 19 conforme au brevet français No 2 704 760 déposé le 4 mai 1993. Le fond 20 de cet embout nasal 19 comporte alors un perçage 21 pour recevoir l'extrémité 14 du conduit secondaire 13 et constituer un élément de maintien de l'extrémité 14 dans l'embout nasal 3.

Le corps 1 peut avoir une autre forme et par exemple : le tronçon coudé 2.3 peut définir un angle obtus ; la tétine 10 peut être dans l'axe de l'extrémité 2.1 ou sur la bissectrice de l'angle formé par le tronçon coudé 2.3... Le conduit secondaire peut en outre être fait en une seule pièce avec le corps 1 ou tout au moins être solidaire de celui-ci.

Bien que l'appareil ait été décrit en relation avec une utilisation sur des personnes, l'appareil est également utilisable dans le domaine vétérinaire.

## Revendications

1. Appareil pour moucher notamment un individu, comprenant un corps (1) délimitant un conduit principal (2) ayant une extrémité pourvue d'un embout nasal (3) et une extrémité opposée pourvue d'un moyen d'aspiration (5), l'appareil comprenant un conduit additionnel (13) ayant une première extrémité (14) débouchant dans l'embout nasal et une deuxième extrémité (15) en communication avec un réservoir (7) de liquide fonctionnel monté sur le corps et pourvu de moyens de propulsion du liquide fonctionnel depuis ledit réservoir jusqu'à l'embout nasal, **caractérisé en ce que** le conduit principal (2) comporte entre ses deux extrémités un tronçon coudé sur une portion externe duquel est monté le réservoir, et **en ce que** le réservoir est agencé pour présenter un volume variable pour permettre une propulsion du liquide fonctionnel depuis ledit réservoir jusqu'à l'embout nasal.

2. Appareil selon la revendication 1, **caractérisé en ce que** le réservoir (7) est conformé en soufflet et comporte un orifice de sortie qui est disposé selon un axe central du soufflet et qui est en communication avec la deuxième extrémité (15) du conduit additionnel (13).

3. Appareil selon la revendication 1, **caractérisé en ce que** le conduit additionnel (13) s'étend dans le conduit principal (2).

4. Appareil selon la revendication 1, **caractérisé en ce que** le conduit additionnel (13) est formé par un tube rapporté de façon amovible dans le conduit principal (2) .

5. Appareil selon la revendication 4, **caractérisé en ce que** l'embout nasal (3) comprend un élément de maintien (16) de la première extrémité (14) du conduit additionnel (13).

6. Appareil selon la revendication 1, **caractérisé en ce que** le réservoir (7) est monté de façon amovible sur le corps (1).

7. Appareil selon la revendication 6, **caractérisé en ce que** le corps (1) comprend un canal (11) ayant une première extrémité (12) dans laquelle débouche la deuxième extrémité (15) du conduit additionnel (13) et une deuxième extrémité débouchant à l'extérieur du corps par une tétine (10) sur laquelle est emmanchée à force une extrémité ouverte (9) du réservoir (7) .

8. Appareil selon la revendication 1, **caractérisé en ce que** le moyen d'aspiration comprend un tuyau souple (5) ayant une extrémité raccordée au conduit principal (2) et une extrémité opposée raccordée à un embout buccal d'aspiration (6, 18).

9. Appareil selon la revendication 8, **caractérisé en ce que** l'embout d'aspiration (18) est réalisé en plastique rigide ou semi-rigide et est agencé pour être serré entre les dents d'un utilisateur, un bourrelet (24) venant en butée de la face arrière des dents.

10. Appareil selon la revendication 1, **caractérisé en ce que** l'extrémité (14) du conduit additionnel (13) débouchant dans l'embout nasal (3) a une position en retrait par rapport à l'orifice de l'embout nasal.

## Claims

1. An appliance for cleaning the nose, in particular of an individual, the appliance comprising a body (1) defining a main duct (2) having one end provided with a nosepiece (3) and an opposite end provided with suction means (5), the appliance including an additional duct (13) having a first end (14) opening out into the nosepiece and a second end (15) in communication with a functional liquid reservoir (7) mounted on the body and provided with means for propelling functional liquid from said reservoir to the nosepiece, the appliance being **characterized in that** between its two ends, the main duct (2) includes a segment with a bend having the reservoir mounted on an external portion thereof, and **in that** the reservoir is arranged to present a volume that is variable to enable the functional liquid to be propelled from said reservoir to the nosepiece.

2. An appliance according to claim 1, **characterized in that** the reservoir (7) is shaped as a bellows and includes an outlet orifice that is disposed on a central axis of the bellows and that is in communication with the second end (15) of the additional duct (13).

3. An appliance according to claim 1, **characterized in that** the additional duct (13) extends inside the main duct (2).

4. An appliance according to claim 1, **characterized in that** the additional duct (13) is formed by a tube removably fitted inside the main duct (2).

5. An appliance according to claim 4, **characterized in that** the nosepiece (3) includes a holder element (16) for holding the first end (14) of the additional duct (13).

6. An appliance according to claim 1, **characterized in that** the reservoir (7) is removably mounted on the body (1).

7. An appliance according to claim 6, **characterized in that** the body (1) includes a channel (11) having a first end (12) in which the second end (15) of the additional duct (13) opens out, and a second end opening out to the outside of the body via a nipple (10) on which an open end (9) of the reservoir (7) is engaged by force.

8. An appliance according to claim 1, **characterized in that** the suction means comprise a flexible hose (5) having one end connected to the main duct (2) and an opposite end connected to a suction mouthpiece (6, 18).

9. An appliance according to claim 8, **characterized in that** the suction mouthpiece (18) is made of rigid or semi-rigid plastic and is arranged to be clamped between the teeth of a user, a rim (24) coming into abutment against the rear face of the teeth.

10. An appliance according to claim 1, **characterized in that** the end (14) of the additional duct (13) opening out into the nosepiece (3) has a position that is set back relative to the orifice of the nosepiece.

## Patentansprüche

1. Vorrichtung zum Naseputzen insbesondere einer Einzelperson, umfassend ein Gehäuse (1), das einen Hauptkanal (2) begrenzt, der ein Ende hat, das mit einem Nasenstöpsel (3) versehen ist, sowie ein entgegengesetztes Ende, das mit Absaugmitteln (5) versehen ist, wobei die Vorrichtung einen zusätzlichen Kanal (13) umfasst, der ein erstes Ende (14) hat, das in den Nasenstöpsel mündet, sowie ein zweites Ende (15), das mit einem Behälter (7) für eine funktionelle Flüssigkeit in Verbindung steht, der an dem Gehäuse angebracht und mit Antriebsmitteln zum Antrieb der funktionellen Flüssigkeit von dem genannten Behälter bis zum Nasenstöpsel versehen ist, **dadurch gekennzeichnet, dass** der Hauptkanal (2) zwischen seinen beiden Enden einen gebogenen Abschnitt umfasst, wobei an einem Außenabschnitt desselben der Behälter angebracht ist, und dass der Behälter so ausgebildet ist, dass er ein veränderliches Volumen aufweist, um einen Antrieb der funktionellen Flüssigkeit von dem genannten Behälter bis zum Nasenstöpsel zu ermöglichen.

2. Vorrichtung nach Anspruch 1, **dadurch gekennzeichnet, dass** der Behälter (7) wie ein Faltenbalg geformt ist und eine Austrittsöffnung hat, die auf einer zentralen Achse des Faltenbalgs angeordnet ist und die mit dem zweiten Ende (15) des zusätzlichen Kanals (13) in Verbindung steht.

3. Vorrichtung nach Anspruch 1, **dadurch gekennzeichnet, dass** sich der zusätzliche Kanal (13) im Hauptkanal (2) erstreckt.

4. Vorrichtung nach Anspruch 1, **dadurch gekennzeichnet, dass** der zusätzliche Kanal (13) aus einem Röhrchen gebildet ist, das lösbar in dem Hauptkanal (2) befestigt ist.

5. Vorrichtung nach Anspruch 4, **dadurch gekennzeichnet, dass** der Nasenstöpsel (3) ein Halteelement (16) zum Halten des ersten Endes (14) des zusätzlichen Kanals (13) umfasst.

6. Vorrichtung nach Anspruch 1, **dadurch gekennzeichnet, dass** der Behälter (7) lösbar am Gehäuse (1) angebracht ist.

7. Vorrichtung nach Anspruch 6, **dadurch gekennzeichnet, dass** das Gehäuse (1) einen Kanal (11) umfasst, der ein erstes Ende (12) hat, in das das zweite Ende (15) des zusätzlichen Kanals (13) mündet, sowie ein zweites Ende, das außerhalb des Gehäuses in einem Anschlussstutzen (10) mündet, auf den mit Kraft ein offenes Ende (9) des Behälters (7) aufgedrückt ist.

8. Vorrichtung nach Anspruch 1, **dadurch gekennzeichnet, dass** die Ansaugmittel einen weichen Schlauch (5) umfassen, der ein Ende hat, das mit der Hauptleitung (2) verbunden ist, sowie ein entgegengesetztes Ende, das mit einem Saugmundstück (6, 18) verbunden ist.

9. Vorrichtung nach Anspruch 8, **dadurch gekennzeichnet, dass** das Saugstück (18) aus einem starren oder halbstarren Kunststoff hergestellt und so ausgebildet ist, dass es mit den Zähnen eines Benutzers festgehalten wird, wobei ein Wulst (24) an der Rückseite der Zähne zur Anlage kommt.

10. Vorrichtung nach Anspruch 1, **dadurch gekennzeichnet, dass** das Ende (14) des zusätzlichen Kanals (13), das in den Nasenstöpsel (3) mündet, eine gegenüber der Öffnung des Nasenstöpsels zurückgesetzte Position hat.
